# EUROPÄISCHE PATENTSCHRIFT

(11) **EP 0 336 100 B1**
(45) Veröffentlichungstag und Bekanntmachung des Hinweises auf die Patenterteilung: **15.09.1993**
(21) Anmeldenummer: 89103271.6
(22) Anmeldetag: 24.02.1989
(51) Int. Cl.: A61B 17/58

(54) **Refixationsstift**
Refixation nail
Clou de refixation

(30) Priorität: 02.04.1988 DE 8804455 U
(43) Veröffentlichungstag der Anmeldung: 11.10.1989
(73) Patentinhaber: AESCULAP AG, D-78501 Tuttlingen (DE)
(72) Erfinder: Beiter, Werner, D-7735 Dauchingen (DE); Lutze, Theodor, D-7201 Balgheim (DE)
(74) Vertreter: Hoeger, Stellrecht & Partner

(56) Entgegenhaltungen:
- FR-A- 2 049 958
- FR-A- 2 256 746
- US-A- 3 977 398

## Beschreibung

Die Erfindung betrifft einen Refixationsstift zum gegenseitigen Festlegen von Knochenteilen, der im Querschnitt quadratisch ist, gebrochene Kanten aufweist und auf jeder Seitenfläche eine radial vorstehende, parallel zu seiner Längsachse verlaufende Rippe trägt, die in Längsrichtung unterbrochen ist.

Für die Refixation von Knochen- und Korpelfragmenten werden überwiegend metallische Implantate eingesetzt. Diese metallischen Implantate finden entweder in Form von sogenannten Kirschnerdrähten oder in Form von Knochenschrauben Verwendung, die die gewünschte Refixation zuverlässig bewerkstelligen. Diese metallischen Implantate haben jedoch den Nachteil, daß sie nach der Einheilung der Fragmente wieder entfernt werden müssen. Es ist also eine weitere Operation notwendig.

Es ist auch bereits bekannt, zur Refixation nagelartige Stifte aus resorbierbaren Kunststoffmaterialien zu verwenden. Diese Stifte werden in die zu fixierenden Knochen- oder Knorpelteile eingesetzt und im Verlauf des Heilungsprozesses vom Körper resorbiert, so daß die Notwendigkeit der nachträglichen Entfernung dieser Implantate entfällt. Nachteilig ist jedoch, daß die bisher verwendeten Stifte nur geringe Kräfte zwischen benachbarten Knochenteilen übertragen können, da die glattwandigen Stifte nach dem Einsetzen in die Knochenteile nicht zuverlässig gegen Verdrehung und Verschiebung gesichert werden können.

Aus der FR-A-2 049 958 ist eine Intramedulärschiene bekannt, die im Markraum eines Röhrenknochens verspannt wird. Dazu wird ein Konus in ein Ende der Schiene eingesetzt und mit Hilfe eines Drehstiftes, der die Schiene der Länge nach durchsetzt, eingeschraubt; dies hat zur Folge, daß sich der Endbereich der Schiene aufspreizt und gegen die Wand des Markraumes drückt. Die im Querschnitt quadratisch ausgebildete Schiene weist gebrochene Kanten auf und trägt auf jeder Seitenfläche eine radial vorstehende, parallel zur Längsrichtung verlaufende Rippe, die in Längsrichtung unterbrochen ist.

Der Erfindung liegt die Aufgabe zugrunde, einen gattungsgemäßen Refixationsstift so auszubilden, daß er besser geeignet ist, Knochen- oder Knorpelfragmente dauerhaft relativ zueinander zu fixieren. Diese Aufgabe wird bei einem Refixationsstift der eingangs beschriebenen Art erfindungsgemäß dadurch gelöst, daß die Außenkanten der Rippen gebrochen sind, daß die gebrochenen Außenkanten des Stiftes und die gebrochenen Kanten der Rippen auf einem gemeinsamen Querschnittskreis liegen und daß die Zwischenräume benachbarter, unterbrochener Rippen in Stiftlängsrichtung gegeneinander versetzt sind.

Ein solcher Stift hat somit einen im wesentlichen sternförmigen Querschnitt. Beim Einsetzen in eine Bohrung mit kreisförmigem Querschnitt wird deren Durchmesser üblicherweise so gewählt, daß der Innendurchmesser der Bohrung dem Kerndurchmesser des Stiftes entspricht, so daß sich die über diesen Kerndurchmesser vorstehenden Rippen und Kanten in die Bohrungswand einarbeiten und somit eine ausgezeichnete Sicherung des Stiftes im Knochengewebe gewährleisten.

Die Rippen drücken sich in das umgebende Knochengewebe ein und verhindern eine Verdrehung und in gewissem Umfange auch eine Längsverschiebung des Stiftes im Knochengewebe. Das umgebende Knochengewebe wächst in die Zwischenräume zwischen die Rippen ein und führt dadurch eine wesentlich bessere Verankerung des Stiftes im Knochen herbei, als dies bei glattwandigen Stiften möglich ist.

Da die Rippen in Längsrichtung unterbrochen sind, ergibt sich eine besonders gute Sicherung. In die Zwischenräume zwischen den in Längsrichtung unterbrochenen Rippen tritt ebenfalls Knochengewebe ein, so daß eine Fixierung des Refixationsstiftes nicht nur gegen Drehung sondern auch gegen Längsverschiebung erreicht werden kann.

Die Fixierung des Refixationsstiftes ist besonders wirksam, wenn die Rippen an den Unterbrechungsstellen scharfkantig enden, da sie dann bereits mit dem Einsetzen eine gewisse Verankerung gegen Längsverschiebung erfahren.

Bei einer besonders vorteilhaften Ausführungsform der Erfindung ist vorgesehen, daß die Rippen im Querschnitt dreieckförmig sind.

Um das Einsetzen zu erleichtern, wird vorgesehen, daß der Stift an einem Ende zugespitzt ist. Außerdem ist es vorteilhaft, wenn der Stift an einem Ende eine kopfförmige Verbreiterung aufweist, die einen Anschlag bildet, der ein zu weites Einschieben des Stiftes in eine Bohrung verhindert.

Die nachfolgende Beschreibung einer bevorzugten Ausführungsform der Erfindung dient im Zusammenhang mit der Zeichnung der näheren Erläuterung. Es zeigen:

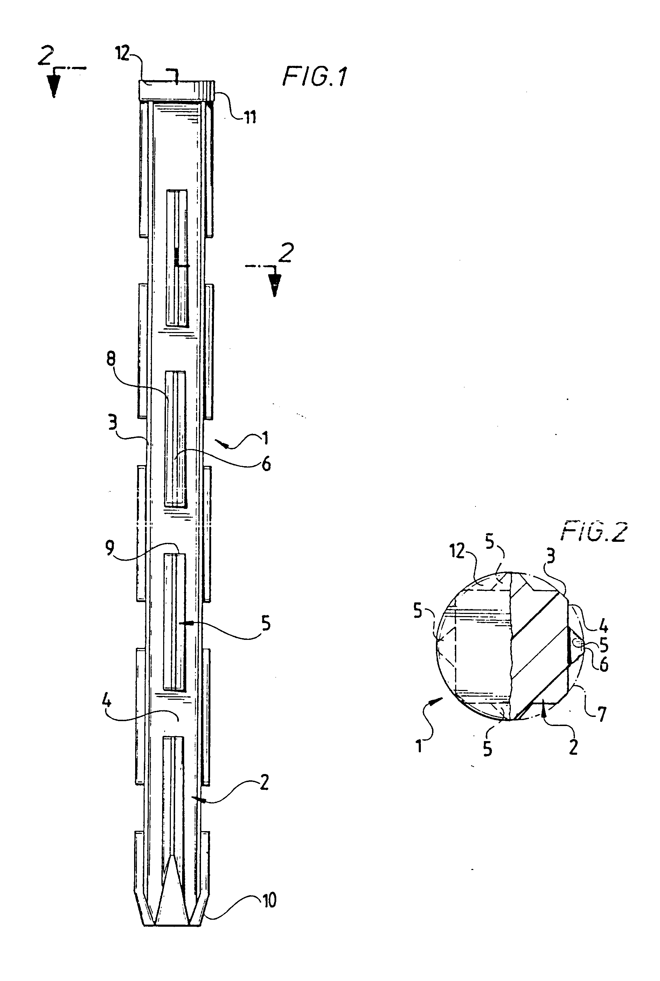

- Fig. 1:: eine Seitenansicht eines erfindungsgemäßen Refixationsstiftes und
- Fig. 2:: eine Schnittansicht längs Linie 2-2 in Fig. 1
Der in der Zeichnung dargestellte Refixationsstift besteht aus einem resorbierbaren Kunststoffmaterial, beispielsweise aus Polylactid.

Dieser Stift 1 weist einen im Querschnitt quadratischen, länglichen Schaft 2 auf, dessen Kanten 3 gebrochen sind. Jede Seitenfläche 4 trägt eine im Querschnitt dreieckförmige, mittig angeordnete, parallel zur Längsachse des Schaftes 2 verlaufende Rippe 5, deren Außenkante 6 ebenfalls gebrochen ist. Dabei verlaufen die gebrochenen Kanten 3 des Schaftes 2 und die gebrochenen Kanten 6 der Rippen 5 auf einem gemeinsamen Querschnittskreis 7, so daß sich insgesamt ein etwa sternförmiger Querschnitt des Stiftes ergibt, wie dies aus der Darstellung der Fig. 2 ersichtlich ist.

Die Rippen 5 sind in Längsrichtung unterbrochen, zwischen benachbarten Rippenstücken 8 befindet sich jeweils ein Zwischenraum 9. Die Rippenstücke 8 enden dabei an den Zwischenräumen 9 scharfkantig, beispielsweise rechtwinklig. Die Zwischenräume 9 in benachbarten Rippen 5 sind in Längsrichtung gegeneinander versetzt.

An einem Ende 10 ist der Stift 1 angespitzt, am gegenüberliegenden Ende 11 trägt er einen tellerförmigen Kopf 12 mit kreisförmigem Querschnitt, dessen Radius mit dem Radius des Querschnittskreises 7 zusammenfällt.

Ein solcher Stift kann beispielsweise eine Länge von 5 cm und einen Kerndurchmesser von 3 mm aufweisen, wobei der Durchmesser des Querschnittskreises 7 zwischen 3,5 und 5 mm liegt.

## Patentansprüche

1. Refixationsstift zum gegenseitigen Festlegen von Knochenteilen, der im Querschnitt quadratisch ist, gebrochene Kanten aufweist und auf jeder Seitenfläche (4) eine radial vorstehende, parallel zu seiner Längsachse verlaufende Rippe (5) trägt, die in Längsrichtung unterbrochen ist,
**dadurch gekennzeichnet,**
daß die Außenkanten (6) der Rippen (5) gebrochen sind, daß die gebrochenen Außenkanten (3) des Stiftes (1) und die gebrochenen Kanten (6) der Rippen (5) auf einem gemeinsamen Querschnittskreis (7) liegen und daß die Zwischenräume (9) benachbarter, unterbrochener Rippen (5) in Stiftlängsrichtung gegeneinander versetzt sind.

2. Stift nach Anspruch 1, dadurch gekennzeichnet, daß die Rippen (5) an den Unterbrechungsstellen scharfkantig enden.

3. Stift nach einem der voranstehenden Ansprüche, dadurch gekennzeichnet, daß die Rippen (5) im Querschnitt dreieckförmig sind.

4. Stift nach einem der voranstehenden Ansprüche, dadurch gekennzeichnet, daß der Stift (1) an einem Ende (10) zugespitzt ist.

5. Stift nach einem der voranstehenden Ansprüche, dadurch gekennzeichnet, daß der Stift (1) an einem Ende (11) eine kopfförmige Verbreiterung (12) aufweist.

## Claims

1. A refixation pin for the fastening of bone parts to one another, which is quadrate in cross-section, has chamfered edges and bears on each lateral surface (4) a radially projecting rib (5) running parallel to its longitudinal axis and being discontinuous in the longitudinal direction, characterised in that the outer edges (6) of the ribs (5) are chamfered, in that the chamfered outer edges (3) of the pin (1) and the chamfered edges (6) of the ribs (5) lie on a common cross-sectional circle, and in that the gaps (9) of neighbouring, discontinuous ribs (5) are staggered in the longitudinal direction of the pin.

2. A pin in accordance with Claim 1, characterised in that the ribs (5) end in sharp edges at the discontinuation sites.

3. A pin in accordance with any one of the preceding Claims, characterised in that the ribs (5) are triangular in cross-section.

4. A pin in accordance with any one of the preceding Claims, characterised in that the pin (1) is tapered at one end (10).

5. A pin in accordance with any one of the preceding Claims, characterised in that one end (11) of the pin (1) has a head-shaped enlargement (12).

## Revendications

1. Clou de refixation pour la fixation réciproque de partie d'os, qui est de section droite carrée, qui présente des arêtes tronquées et qui porte sur chaque surface latérale (4) une nervure (5) qui fait saillie radialement, qui s'étend parallèlement à son axe longitudinal et qui est interrompue en direction longitudinale, caractérisé en ce que les arêtes externes (6) des nervures (s) sont tronquées, en ce que les arêtes externes tronquées (3) du clou (1) et les arêtes tronquées (6) des nervures (5) sont situées sur un cercle en section droite (7) commun et en ce que les interstices (9) situés entre des nervures interrompues (5) voisines sont décalés les uns par rapport aux autres dans la direction longitudinale du clou.

2. Clou selon la revendication 1, caractérisé en ce que les nervures (5) se terminent par des arêtes vives au niveau des points d'interruption.

3. Clou selon l'une des revendications précédentes, caractérisé en ce que les nervures (5) sont de section droite triangulaire.

4. Clou selon l'une des revendications précédentes, caractérisé en ce que le clou (1) se termine en pointe à une extrémité (10).

5. Clou selon l'une des revendications précédentes, caractérisé en ce que le clou (1) comporte à une extrémité (11) un élargissement en forme de tête (12).
